# EUROPÄISCHE PATENTSCHRIFT

(11) **EP 1 453 427 B1**
(45) Veröffentlichungstag und Bekanntmachung des Hinweises auf die Patenterteilung: **30.08.2006**
(21) Anmeldenummer: 02791629.5
(22) Anmeldetag: 16.12.2002
(51) Int. Cl.: A61B 17/66, A61C 13/00

(54) **VORRICHTUNG ZUM KNOCHENAUFBAU DURCH DISTRAKTION VON KNOCHENSEGMENTEN**
DEVICE FOR BONE FORMATION BY DISTRACTION OF BONE SEGMENTS
DISPOSITIF POUR L'OSTEOGENESE PAR DISTRACTION DE SEGMENTS OSSEUX

(30) Priorität: 15.12.2001 DE 10161691
(43) Veröffentlichungstag der Anmeldung: 08.09.2004
(73) Patentinhaber: Richard Blankenhorn Gmbh, 73734 Esslingen (DE)
(72) Erfinder: BLANKENHORN, Ulrich, 73734 Esslingen (DE); KOLLROSS, Rainer, 73663 Berglen (DE); SCHWEIZER, Wolfgang, 71522 Backnang (DE)
(74) Vertreter: Herzog, Günter
(86) Internationale Anmeldenummer: PCT/DE2002/004601
(87) Internationale Veröffentlichungsnummer: WO 2003/051220

(56) Entgegenhaltungen:
- WO-A-00/21455
- DE-C- 19 848 599
- DE-U- 29 919 487
- US-A- 5 791 900

## Beschreibung

### Stand der Technik

Die Erfindung geht aus von einer Vorrichtung zur Distraktion von Knochensegmenten.

Die Knochendistraktion dient zum gezielten Aufbau von Knochenmaterial, indem ein Teil des Kieferknochens abgetrennt und über einen längeren Zeitraum angehoben wird, bis in etwa die Höhe des ursprünglich vorhandenen Kieferkammes erreicht wird. In diesem so entstehenden künstlichen Spalt wächst bei dem als Osteogenese bekannten Vorgang durch gewollte Einblutungen ein Kallusgewebe nach, das im Laufe von wenigen Monaten zu reifem Knochen heranwächst. In dieses Knochenmaterial lässt sich ein Zahnimplantat einsetzen.

Aus der Patentschrift DE 198 48 599 C1 ist eine Vorrichtung zur Distraktion eines Kieferknochens bekannt, bei der als Brückenelement ein hinreichend stabiler Draht verwendet wird. Diese Vorrichtung hat den Nachteil, dass durch die Verankerungseinrichtung an dem abgetrennten Knochenteil ein Abwandern dieses Knochenteils zur Gaumen- oder Zungenseite hin stattfinden kann. Außerdem wird der Bereich der Zahnlücke nicht geschützt, wodurch der Aufbau des Knochens durch Verletzungen beeinträchtigt wird.

Aus der Gebrauchmusterschrift DE 299 19 487 U1 ist ein Knochendistraktor im Bereich des Alveolarknochens bekannt, der aus einer Verstellspindel mit einem Lagerkopf und aus einer mit der Verstellspindel kämmenden Knochenplatte, die über eine Knochenschraube mit dem zu distrahierenden Knochenteil des Alveolarknochens verschraubbar ist, besteht. Der Nachteil dieses "Korkenziehers" ist, dass die Vorrichtung für den Patienten unangenehm groß ist. Ein weiterer Nachteil dieser Vorrichtung ist, dass die empfindlichen anatomischen Strukturen des Alveolarfortsatzes durch die Verankerung im Alveolarknochen geschädigt werden können. Gerade der basale Anteil des Alveolarfortsatzes enthält Strukturen des menschlichen Gesichtsschädels, die möglichst nicht angetastet werden sollten. Solche Strukturen sind im Unterkiefer der Mandibularkanal mit Nerven- und Blutgefäßen und im Oberkiefer die Nasen- sowie Kieferhöhle.

### Die Erfindung und ihre Vorteile

Die erfindungsgemäße Vorrichtung mit den kennzeichnenden Merkmalen des Hauptanspruches hat demgegenüber den Vorteil, dass als Trageinrichtung ein festsitzendes und ästhetisch ansprechendes Langzeitprovisorium während der Wachstumsphase des Kieferknochens verwendet wird, das ein Abwandern des zu distrahierenden Segments zur Gaumen-oder Zungenseite hin verhindert.

Dadurch, dass das Widerlager der Distraktionsschraube nicht im basalen Anteil des Alveolarfortsatzes, sondern am Langzeitprovisorium befestigt ist, werden die empfindlichen anatomischen Strukturen dieses Knochens geschont. Die Operationsebene wird auf eine höhere Ebene verlegt, daher ist eine geringere Restknochenhöhe notwendig.

Das Langzeitprovisorium wird entsprechend der individuellen Gebisssituation des Patienten im zahntechnischen Labor so hergestellt, dass es die Zahnlücke abdeckt.

Das Langzeitprovisorium ist zahnähnlich aufgebaut und/oder mit einem Kaurelief versehen und deckt eine zahnlücke ab.

Nach einer anderen vorteilhaften Ausgestaltung der Erfindung ist das Langzeitprovisorium im Bereich der oralen Fläche des zu distrahierenden Knochensegments zusätzlich mit einer Leitschiene für das mobile Knochensegment versehen.

Das Langzeitprovisorium ist in Form einer Brücke gestaltet.

Nach einer anderen vorteilhaften Ausgestaltung der Erfindung wird das Langzeitprovisorium an den natürlichen Zähnen, an Implantaten oder an einer Kombination aus Zähnen und Implantaten im Mund des Patienten befestigt.

Nach einer vorteilhaften Ausgestaltung der Erfindung ist das Langzeitprovisorium nach dem Prinzip der verlorenen Wachsmodellierung aus einer Co-Cr-Mo-Legierung gegossen. Als Abformmaterialien dienen Alginat oder Hydrokolloid.

Nach einer anderweitigen vorteilhaften Ausgestaltung der Erfindung ist das Langzeitprovisorium mit einer zahnfarbenen Kunststoffverblendung überzogen.

Nach einer zusätzlichen vorteilhaften Ausgestaltung der Erfindung besitzt die Knochenbuchse ein mit einer angerauten Oberfläche versehenes Außengewinde und ein komplett durchgängiges, dem Außengewinde des Distraktionsmittels komplementäres Innengewinde.

Nach einer anderen vorteilhaften Ausgestaltung der Erfindung Kann die Distraktionsschraube zwischen dem Langzeitprovisorium und der derben Schleimhaut fixiert werden. Durch einen Winkel der Knochenbuchse zum Zylinder wird ein Kraftvektor aufgebaut, der die derbe Schleimhaut anhebt und gleichzeitig die Versorgung zum Knochenaufbau erhält.

Weitere Vorteile und vorteilhafte Ausgestaltungen der Erfindung sind der nachfolgenden Beschreibung, der Zeichnung und den Ansprüchen entnehmbar.

### Zeichnung

Ein Ausführungsbeispiel der Erfindung ist in den Zeichnungen dargestellt und im Folgenden näher beschrieben.
- Fig. 1:: Darstellung der Zahnlücke als Ausgangssituation;
- Fig. 2:: Darstellung der Zahnlücke mit gebohrtem Loch in dem vom Kieferknochen getrennten mobilen Knochensegment;
- Fig. 3:: Darstellung der Zahnlücke, nachdem in das gebohrte Loch eine Knochenbuchse mit Innengewinde und selbstschneidendem Außengewinde eingebracht wurde;
- Fig. 4:: Darstellung der Zahnlücke, nachdem diese mit einem Langzeitprovisorium überbrückt wurde;
- Fig. 5:: Darstellung der Zahnlücke mit eingeschraubter Distraktionsschraube;
- Fig. 6:: Darstellung der Zahnlücke mit durch die Distraktionsschraube angehobenem mobilen Knochensegment;
- Fig. 7:: Darstellung eines Querschnitts durch den Kiefer mit dem mobilen Knochensegment.

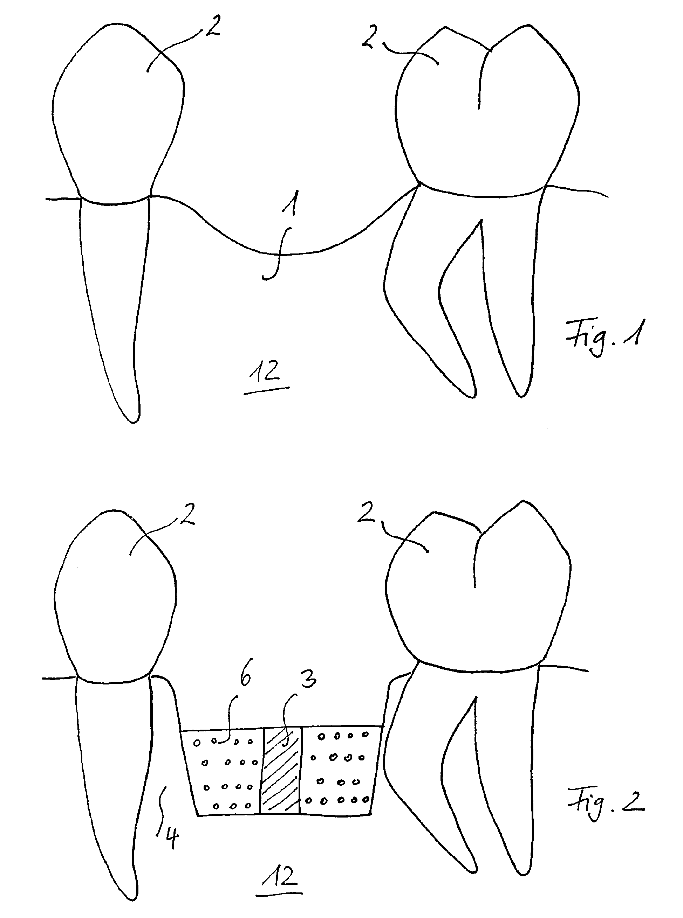

Fig. 1 zeigt eine Zahnlücke 1 zwischen zwei verbliebenen, der Zahnlücke 1 benachbarten Zähnen 2 und die nach etwa einem Jahr seit Fehlen des Zahnes entstandene Vertiefung durch Verlust von Knochengewebe.

In Fig. 2 ist das Einbringen einer Bohrung 3 in den Kieferknochen 4 dargestellt. Diese Bohrung ist üblicherweise kreisrund.

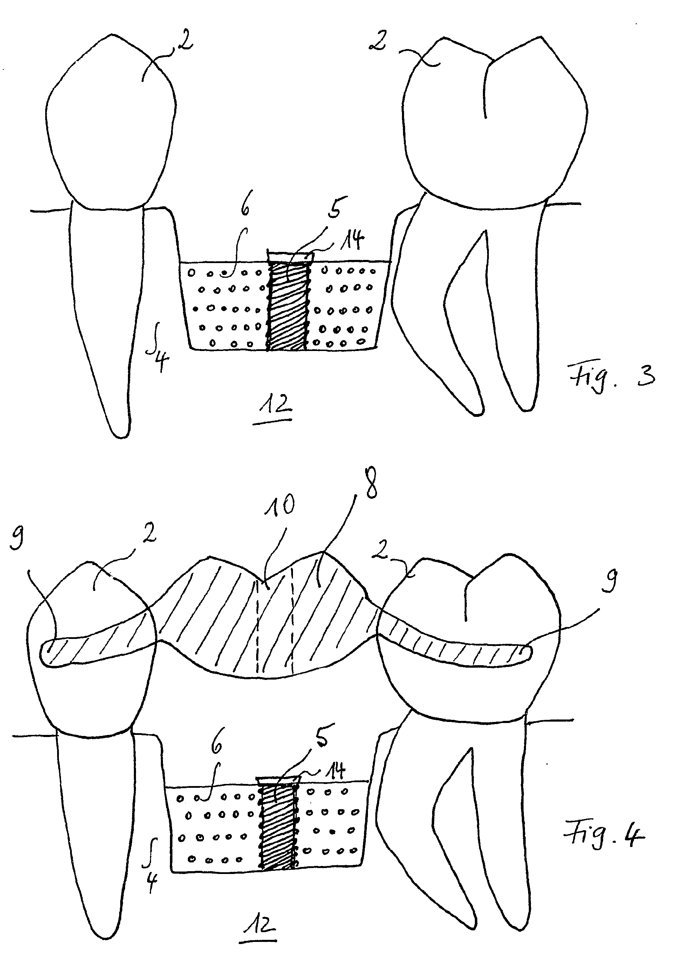

Fig. 3 zeigt die Verankerung der Knochenbuchse 5 im zu distrahierenden und somit mobilen Anteil des Alveolarfortsatzes, des mobilen Knochensegments 6. Durch Drehen der in die Knochenbuchse 5 eingesetzten Distraktionsschraube 7 wird der Trennbereich zwischen den beiden Knochensegmenten vergrößert.

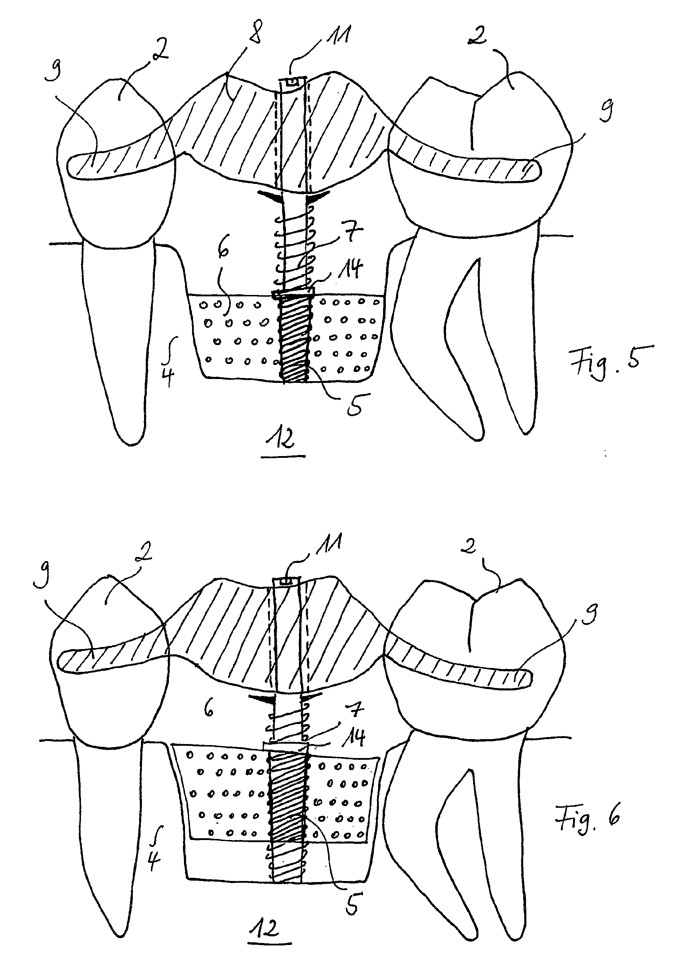

In Fig. 4 wird dargestellt, wie die mit der Knochenbuchse 5 und der Distraktionsschraube 7 versehene Zahnlücke 1 mit dem Langzeitprovisorium 8 überbrückt wird. Das Langzeitprovisorium 8 wird mit den Befestigungsmitteln 9 an den Zähnen 2 befestigt. Es enthält zusätzlich eine Bohrung 10 für die Distraktionsschraube 7.

Zum Drehen der Distraktionsschraube 7 wird ein Werkzeugangriff 11 im Kopf der Distraktionsschraube 7 benützt, wie in Fig. 5 dargestellt.

Der Vorgang des Vergrößerns des Zwischenraums zwischen dem mobilen Knochensegment 6 und dem Kieferknochen 4 wird in Fig. 6 dargestellt. Dieser Vorgang erfolgt sukzessive bei regelmäßig erforderlichen Zahnarztbesuchen.

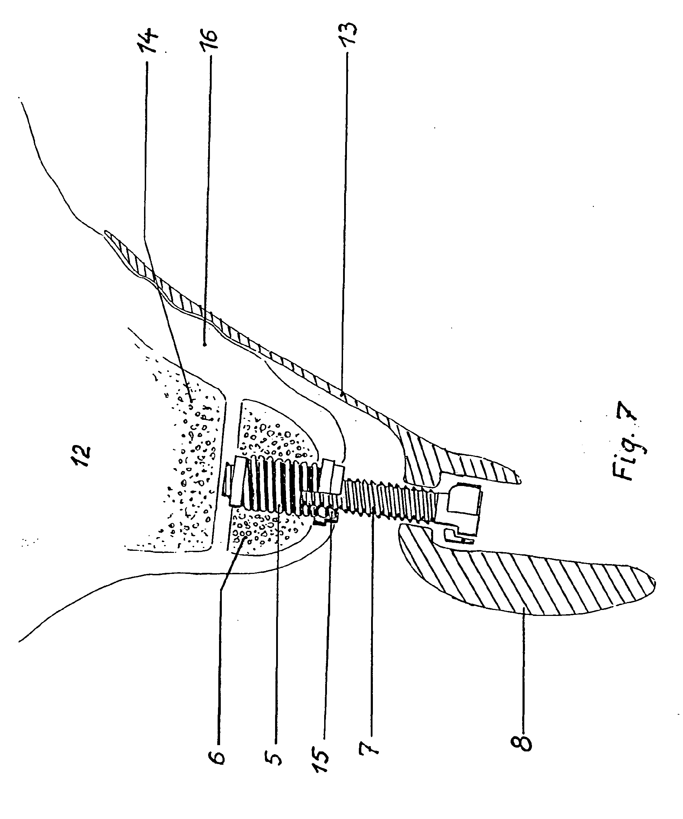

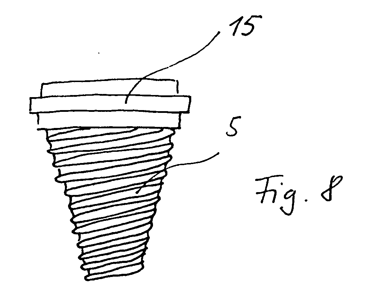

In Fig. 7 läuft die Schnittebene quer zur bisherigen senkrechten Schnittansicht der vorangegangenen Fig. 1 bis 6. Dargestellt ist die Verankerung der Knochenbuchse 5 im mobilen Knochensegment 6 mit der Distraktionsschraube 7 und dem Werkzeugangriff 11 sowie die Befestigungsmittel 9 des Langzeitprovisoriums 8 sowie die Leitschiene 13.

Nach etwa drei Monaten wird das Langzeitprovisorium 8 mit der Distraktionsschraube 7 entfernt und in die Knochenbuchse 5 das endgültige Implantat eingeschraubt, das durch das sekundär nachgewachsene Knochengewebe festen Halt im Kiefer 12 hat. Es ist aber auch möglich, dass vor dem Einsetzen des Implantats die Knochenbuchse ebenfalls entfernt wird.

Das Langzeitprovisorium kann durch Präzisions-Abformmaterialien abgegossen und/oder nach dem Prinzip der verlorenen Wachsmodellation aus einer Co-Cr-Mo-Legierung gegossen werden. Es kann nach dem CAD-CAM-Verfahren hergestellt werden.

Alle in der Beschreibung, den nachfolgenden Ansprüchen und der Zeichnung dargestellten Merkmale können sowohl einzeln als auch in beliebiger Kombination miteinander erfindungswesentlich sein.

### Bezugszahlenliste

- 1:: Zahnlücke
- 2:: Zähne
- 3:: Bohrung
- 4:: Kieferknochen
- 5:: Knochenbuchse
- 6:: mobiles Knochensegment
- 7:: Distraktionsschraube
- 8:: Langzeitprovisorium
- 9:: Befestigungsmittel
- 10:: Bohrung für die Distraktionsschraube
- 11:: Werkzeugangriff im Kopf der Distraktionsschraube
- 12:: Kiefer
- 13:: Leitschiene

## Patentansprüche

1. Vorrichtung zum Knochenaufbau mit einer Knochenbuchse (5), die in ein mobiles Knochensegment (6) eingebracht werden kann, wobei die Knochenbuchse mit einem Distraktionsmittel an einer Trageinrichtung angeordnet ist
**dadurch gekennzeichnet,**
**dass** die Trageinrichtung ein als Brücke ausgebildetes Langzeitprovisorium (8) ist, das zahnähnlich aufgebaut ist und/oder ein Kaurelief besitzt und eine Zahnlücke abdeckten kann.

2. Vorrichtung zum Knochenaufbau nach Anspruch 1
**dadurch gekennzeichnet,**
**dass** das Langzeitprovisorium (8) eine Leitschiene (13) besitzt.

3. Vorrichtung zum Knochenaufbau nach einem der vorhergehenden Ansprüche,
**dadurch gekennzeichnet,**
**dass** das Distraktionsmittel als Schraube (7) ausgebildet ist.

4. Vorrichtung zum Knochenaufbau nach einem der vorhergehenden Ansprüche,
**dadurch gekennzeichnet,**
**dass** das Langzeitprovisorium (8) als Brücke an mindestens zwei Zähnen (2) angeordnet werden kann.

5. Vorrichtung zum Knochenaufbau nach einem der vorhergehenden Ansprüche,
**dadurch gekennzeichnet,**
**dass** das Langzeitprovisorium (8) als Brücke an mindestens einem Zahn und mindestens einem Implantat angeordnet werden kann.

6. Vorrichtung zum Knochenaufbau nach einem der vorhergehenden Ansprüche,
**dadurch gekennzeichnet,**
**dass** das Langzeitprovisorium (8) als Brücke an mindestens zwei Implantaten angeordnet werden kann.

7. Vorrichtung zum Knochenaufbau nach einem der vorhergehenden Ansprüche,
**dadurch gekennzeichnet,**
**dass** das Langzeitprovisorium (8) mit einer zahnfarbenen Verblendung versehen ist.

8. Vorrichtung zum Knochenaufbau nach einem der vorhergehenden Ansprüche,
**dadurch gekennzeichnet,**
**dass** die Knochenbuchse (5) konisch ausgebildet ist.

9. Vorrichtung zum Knochenaufbau nach einem der vorhergehenden Ansprüche,
**dadurch gekennzeichnet,**
**dass** die Knochenbuchse (5) selbstschneidend ausgebildet ist.

10. Vorrichtung zum Knochenaufbau nach einem der vorhergehenden Ansprüchen,
**dadurch gekennzeichnet,**
**dass** die Knochenbuchse (5) in Richtung zum Zahnfleisch (16) einen Kragen (15) besitzt.

11. Vorrichtung zum Knochenaufbau nach einem der vorhergehenden Ansprüche,
**dadurch gekennzeichnet,**
**dass** die Knochenbuchse (5) ein Außengewinde mit einer angerauten Oberfläche besitzt.

## Claims

1. A device for bone synthesis having a bone bushing (5) which can be inserted into a mobile bone segment (6), whereby the bone bushing is arranged with a distraction means on a supporting device,
**characterized in that**
the supporting device is a long-term temporary part (8) designed as a bridge and having a dental-like composition and/or having a relief of the jaw and optionally covering a tooth gap.

2. The device for bone synthesis according to Claim 1,
**characterized in that**
the long-term temporary part (8) has a guide rail (13).

3. The device for bone synthesis according to any one of the preceding claims,
**characterized in that**
the distraction means is designed as a screw (7).

4. The device for bone synthesis according to any one of the preceding claims,
**characterized in that**
the long-term temporary part (8) may be arranged as a bridge on at least two teeth (2).

5. The device for bone synthesis according to any one of the preceding claims,
**characterized in that**
the long-term temporary part (8) may be arranged as a bridge on at least one tooth and at least one implant.

6. The device for bone synthesis according to any one of the preceding claims,
**characterized in that**
the long-term temporary part (8) may be arranged as a bridge on at least two implants.

7. The device for bone synthesis according to any one of the preceding claims,
**characterized in that**
the long-term temporary part (8) is provided with a tooth-colored veneer.

8. The device for bone synthesis according to any one of the preceding claims,
**characterized in that**
the bone bushing (5) is designed to be conical.

9. The device for bone synthesis according to any one of the preceding claims,
**characterized in that**
the bone bushing (5) is designed to be self-cutting.

10. The device for bone synthesis according to any one of the preceding claims,
**characterized in that**
the bone bushing (5) has a collar (15) in the direction of the gingiva (16).

11. The device for bone synthesis according to any one of the preceding claims,
**characterized in that** the bone bushing (5) has an outside thread with a roughened surface.

## Revendications

1. Dispositif d'ostéogénèse, comportant une broche à os (5) qui peut être intégrée dans un segment osseux mobile (6), la broche à os étant disposée à l'aide d'un moyen de distraction sur un dispositif support,
**caractérisé en ce que**
le dispositif porteur est un appareil provisoire de longue durée (8) réalisé en forme de pont qui est conçu à la manière de dents et/ou présente un relief de mâchoire et peut recouvrir un intervalle entre des dents.

2. Dispositif d'ostéogénèse selon la revendication 1,
**caractérisé en ce que**
l'appareil provisoire de longue durée (8) comporte un rail conducteur (13).

3. Dispositif d'ostéogénèse selon une des revendications précédentes,
**caractérisé en ce que**
le moyen de distraction se présente sous la forme d'une vis (7).

4. Dispositif d'ostéogénèse selon une des revendications précédentes,
**caractérisé en ce que**
l'appareil provisoire de longue durée (8) peut être disposé en forme de pont sur au moins deux dents (2).

5. Dispositif d'ostéogénèse selon une des revendications précédentes,
**caractérisé en ce que**
l'appareil provisoire de longue durée (8) peut être disposé en forme de pont sur au moins une dent et au moins un implant.

6. Dispositif d'ostéogénèse selon une des revendications précédentes,
**caractérisé en ce que**
l'appareil provisoire de longue durée (8) peut être disposé en forme de pont sur au moins deux implants.

7. Dispositif d'ostéogénèse selon une des revendications précédentes,
**caractérisé en ce que**
l'appareil provisoire de longue durée (8) est équipé d'un enrobage de la couleur des dents.

8. Dispositif d'ostéogénèse selon une des revendications précédentes,
**caractérisé en ce que**
la broche à os (5) a une forme conique.

9. Dispositif d'ostéogénèse selon une des revendications précédentes,
**caractérisé en ce que**
la broche à os (5) a une forme auto-coupante.

10. Dispositif d'ostéogénèse selon une des revendications précédentes,
**caractérisé en ce que**
la broche à os (5) comporte une collerette (15) en direction de la gencive (6).

11. Dispositif d'ostéogénèse selon une des revendications précédentes,
**caractérisé en ce que**
la broche à os (5) comporte un filetage extérieur à surface rugueuse.
